# EUROPEAN PATENT APPLICATION

(11) **EP 2 184 020 A2**
(43) Date of publication of application: **12.05.2010**
(21) Application number: 09252546.8
(22) Date of filing: 04.11.2009
(51) Int. Cl.: A61B 17/34

(54) **Surgical access device**

(30) Priority: 06.11.2008 US 111842 P; 16.10.2009 US 580637
(71) Applicant: Tyco Healthcare Group LP, North Haven, CT 06473 (US)
(72) Inventor: Fischvogt, Gregory, Hamden, CT 06514 (US)
(74) Representative: Soames, Candida Jane

(57) **Abstract**

The present disclosure relates to a surgical access device for use during a surgical procedure that includes a housing, an access member extending distally from the housing, and a valve that is at least partially disposed within the housing. The access member is dimensioned for positioning within tissue, defines a longitudinal axis and a longitudinal opening, and has proximal and distal ends. The valve has a passage for the reception of a surgical object, and includes a proximal collar segment, a distal tapered segment that extends contiguously from the proximal collar segment, and a fluid resistive shelf. The fluid resistive shelf is generally defined at a juncture of the proximal collar segment and the distal tapered segment. The fluid resistive shelf and the distal tapered segment cooperate to substantially minimize the egress of fluids from the operative site about the valve.

## Description

### CROSS REFERENCE TO RELATED APPLICATIONS

The present application claims the benefit of and priority to U.S. Provisional Application Serial No. 61/111,842, filed on November 6, 2008, the entire contents of which are incorporated herein by reference.

### BACKGROUND

### 1. Technical Field

The present disclosure relates to a surgical access device that is removably positionable within a tissue tract formed in a patient's tissue. More specifically, the present disclosure relates to a surgical access device that includes a valve assembly adapted to accommodate the insertion of surgical objects and/or surgical filaments, while substantially limiting the communication of fluids therethrough.

### 2. Background of the Related Art

Many surgical procedures are performed through access devices, e.g., trocar and cannula assemblies. These devices incorporate narrow tubes or cannulae percutaneously inserted into a patient's body, through which one or more surgical objects may be introduced to access a surgical worksite. Generally, such procedures are referred to as "endoscopic," unless the procedure is related to the examination/treatment of a joint, in which case the procedure is referred to as "arthroscopic", or to the examination/treatment of a patient's abdomen, in which case the procedure is referred to as "laparoscopic."

During these procedures, surgical filaments are often used to repair openings in skin, internal organs, blood vessels, and the like, as in the case of meniscal repair, and to join various tissues together, as in the reattachment of ligaments or tendons to bone. Additionally, a fluid, such as an insufflation gas or saline, is often introduced into the surgical worksite to increase visibility or access to the tissue that is the subject of the procedure. Accordingly, the establishment and maintenance of a substantially fluid-tight seal is desirably to curtail the escape of such fluids and preserve the integrity of the surgical worksite. To this end, surgical access devices generally incorporate a seal through which the surgical object and/or surgical filaments are inserted.

While many varieties of seals are known in the art, there exists a continuing need for a seal that can accommodate a variety of differently-sized surgical objects and/or surgical filaments while substantially limiting the escape of fluids.

### SUMMARY

Accordingly, the present disclosure relates to a surgical access device for use during a surgical procedure. The surgical access device includes a housing, an access member extending distally from the housing and defining a longitudinal opening, and having proximal and distal ends, and a valve at least partially disposed within the housing and having a passage for reception of a surgical object, The valve including a proximal collar segment, a distal tapered segment extending contiguously from the proximal collar segment and a fluid resistive shelf generally defined at a juncture defined by the proximal collar segment and the distal tapered segment. At least the fluid resistive shelf and the distal tapered segment cooperate to substantially minimize egress of fluids from the operative site about the valve.

The valve may be dimensioned whereby the passage is substantially closed in the absence of the surgical object. The distal tapered segment of the valve may define a substantially frusto-conical shape. The valve may include at least one internal slit with the at least one internal slit defining the passage. The valve may include a plurality of intersecting slits with the slits defining the passage.

The proximal collar segment of the valve may include a recessed portion defining an internal wall. The internal wall tapers radially inwardly from proximal to distal to assist in guiding the surgical object towards the passage of the valve. The valve may be a solid member.

An instrument seal may be mounted relative to the housing. The instrument seal defines a seal aperture for reception of the surgical object in substantial sealed relation therewith. At least one of the instrument seal and the valve includes structure configured and dimensioned to maintain the relative position of the instrument seal and the valve. The valve may include a recess configured and dimensioned to engage a corresponding ridge formed on the instrument seal. The valve may include a lip configured and dimensioned to accommodate the instrument seal such that the instrument seal is at least partially positionable within the valve.

A surgical procedure is also disclosed. The surgical procedure may include the steps of:

accessing an operative site with a surgical access device, the surgical access device including an access member defining a longitudinal opening and having proximal and distal ends, and a valve including a proximal collar segment, a distal tapered segment extending contiguously from the proximal collar segment, and a fluid resistive shelf defined at a juncture defined by the proximal collar segment and the distal tapered segment;

introducing fluids into the operative site;

substantially minimizing egress of fluids from the operative site and about the valve through engagement of the fluids with the fluid resistive shelf and the distal tapered segment; and

introducing a surgical object through the valve and through the longitudinal opening of the access member to perform a surgical task.

These and other features of the surgical access device and valve assembly disclosed herein will become more readily apparent to those skilled in the art from the following detailed description of various embodiments of the present disclosure.

The invention may be described by reference to the following numbered paragraphs:-

A surgical access device including an access member defining a longitudinal opening and having proximal and distal ends, and a valve including a proximal collar segment, a distal tapered segment extending contiguously from the proximal collar segment, and a fluid resistive shelf at a juncture defined by the proximal collar segment and the distal tapered segment, for use in a surgical procedure wherein the procedure comprises the steps of:
accessing an operative site with the surgical access device,
introducing fluids into the operative site;
substantially minimizing egress of fluids from the operative site and about the valve through engagement of the fluids with the fluid resistive shelf and the distal tapered segment; and introducing a surgical object through the valve and through the longitudinal opening of the access member to perform a surgical task.

The surgical access device of paragraph [0017] wherein the proximal collar segment of the valve includes a recessed portion defining an internal wall tapering radially inwardly from proximal to distal and wherein the step of accessing includes guiding the surgical object towards the passage of the valve.

The surgical access device of [0018] wherein the access device further includes an object seal mounted relative to the housing and wherein the step of introducing includes establishing a substantial sealed relation about the surgical object with the object seal.

### BRIEF DESCRIPTION OF THE DRAWINGS

Various embodiments of the present disclosure are described herein below with references to the drawings, wherein:

FIG. 1 is a side, schematic view of a surgical access device incorporating one embodiment of a valve assembly including a valve in accordance with the principles of the present disclosure;

FIG. 2 is a side, perspective view of the valve seen in FIG. 1 removed from the surgical access device and prior to the insertion of a surgical object;

FIG. 3 is a side, perspective view of an alternative embodiment of the valve shown in FIG. 2;

FIG. 4 is a top view of the valve shown in FIGS. 1 and 2;

FIG. 5 is a bottom view of the valve shown in FIGS. 1, 2 and 4;

FIG. 6 is a side, perspective view of the valve shown in FIGS. 1, 2, 4, and 5 with a surgical object inserted therethrough;

FIG. 7 is a side, perspective view of an alternative embodiment of the valve shown in FIG. 2 shown prior to the insertion of a surgical object;

FIG. 8 is a side, perspective view of another embodiment of the valve assembly seen in FIG. 1 including an instrument seal positionable proximally of, and illustrated spaced from, the valve shown in FIG. 7 prior to the insertion of a surgical object;

FIG. 9 is a bottom view of an alternative embodiment of the instrument seal seen in FIG. 8;

FIG. 10 is a bottom view of another embodiment of the instrument seal seen in FIG. 8;

FIG. 11 is a side, perspective view of another embodiment of the valve assembly shown in FIG. 8 with parts separated, wherein the valve includes a lip configured and dimensioned to at least partially accommodate the instrument seal, and shown prior to the insertion of a surgical object;

FIG. 12 is a side, perspective view of the valve assembly shown in FIG. 11 illustrating the valve assembled together with the instrument seal; and

FIG. 13 is a side, perspective view of the valve assembly shown in FIG. 8 illustrating the valve and the instrument seal separated from each other with a surgical object inserted therethrough.

### DETAILED DESCRIPTION

In the drawings, and in the following description, in which like reference characters identify similar or identical elements, the term "proximal" should be understood as referring to the end of the disclosed surgical access device, or any component thereof, that is closest to a practitioner during use, while the term "distal" should be understood as referring to the end that is farthest from the practitioner during use. Additionally, the term "surgical object" should be understood as referring to any surgical object or instrument that may be employed during the course of surgical procedure, including but not limited to an obturator, a surgical stapling device, or the like; the term "filament" should be understood as referring to any elongate member suitable for the intended purpose of joining tissue, including but not limited to sutures, ligatures, and surgical tape; and the term "tissue" should be understood as referring to any bodily tissue, including but not limited to skin, fascia, ligaments, tendons, muscle, and bone.

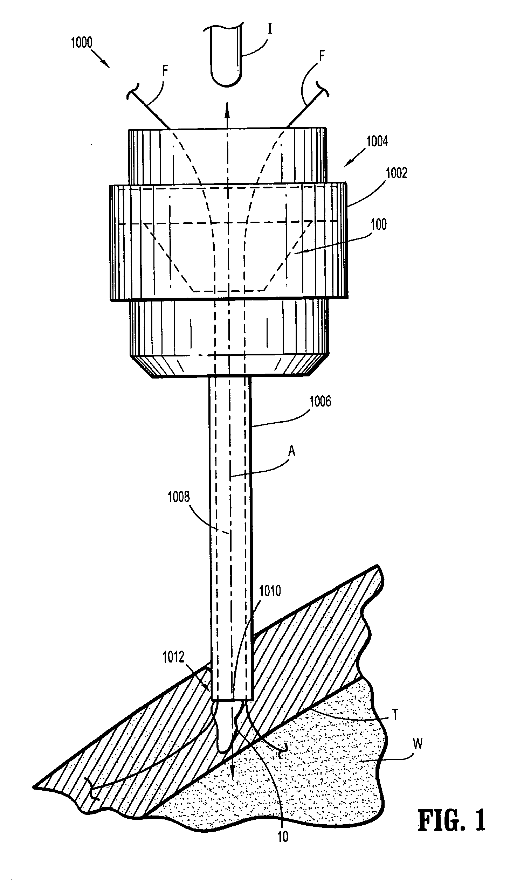

FIG. 1 illustrates a surgical access device 1000 including a housing 1002 at a proximal end 1004 thereof, and an access member 1006 that extends distally from the housing 1002. The housing 1002 is configured and dimensioned to accommodate a valve assembly, one embodiment of which is shown and referred to generally by reference character 100, and may be any structure suitable for this intended purpose.

The access member 1006 is dimensioned for positioning with a percutaneous tissue tract 10 formed in a patient's tissue "T" to provide access to a surgical worksite "W." The access member 1006 defines a longitudinal opening 1008 that extends longitudinally therethrough and defines a longitudinal axis "A." The longitudinal opening 1008 is configured and dimensioned for the internal receipt of one or more surgical objects "I" and/or surgical filaments "F." The access member 1006 defines an opening 1010 at a distal end 1012 thereof to allow the surgical object "I" and/or the surgical filaments "F" to pass therethrough.

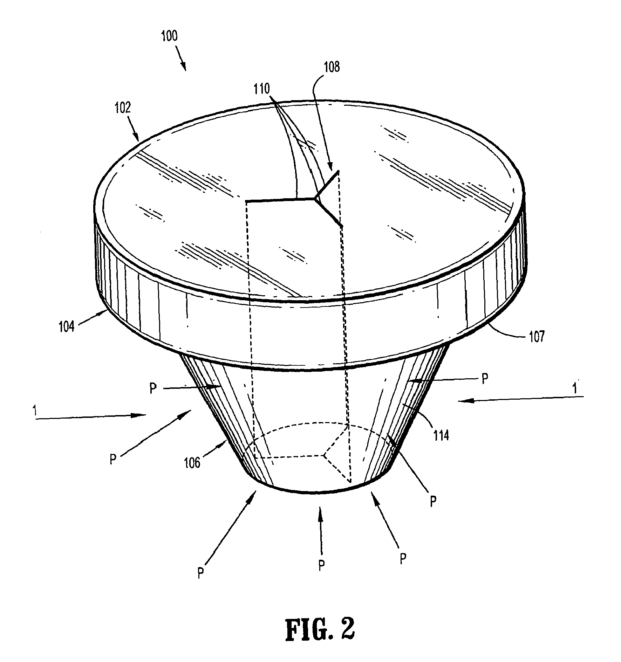

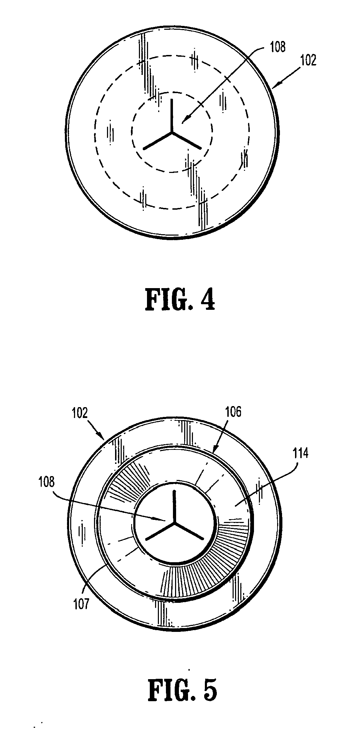

Referring now to FIGS. 2-6 as well, the valve assembly 100 will be discussed. The valve assembly 100 includes a valve 102, which may be formed from any suitable material that is at least semi-resilient in.nature, and fabricated through any suitable method of manufacture, including but not limited to molding, casting, and electrical discharge machining (EDM). Examples of suitable materials include, but are not limited to elastomeric materials such as natural rubber, synthetic polyisoprene, butyl rubber, halogenated butyl rubbers, polybutadiene, styrene-butadiene rubber, nitrile rubber, hydrogenated nitrile rubbers, chloroprene rubber, ethylene propylene rubber, ethylene propylene diene rubber, epichlorohydrin rubber, polyacrylic rubber, silicone rubber, fluorsilicone rubber, fluoroelastomers, perfluoroelastomers, polyether block amides, chlorosulfonated polyethylene, ethylene-vinyl acetate, thermoplastic elastomers, thermoplastic vulcanizers, thermoplastic polyurethane, thermoplastic olefins, resilin, elastin, and polysulfide rubber. Forming the valve 102 from such materials permits the valve 102 to resiliently accommodate the insertion, manipulation, and removal of surgical objects "I" and/or surgical filaments "F" that may vary in size, e.g., outer dimensions. The valve 102 may be either solid, as illustrated in FIGS. 2-6, or alternatively, the valve 102 may include one or more internal spaces.

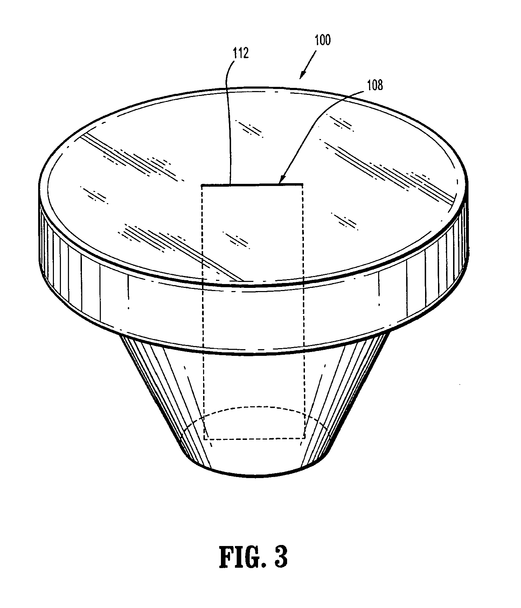

The valve 102 includes a proximal collar segment 104, a distal segment 106 extending contiguously from the proximal collar segment 104, a fluid resistive shelf 107 generally defined at the juncture of the distal segment 106 and the proximal collar segment 104, and a passage 108 for reception of the surgical object "I" and/or surgical filaments "F" (FIG. 1). The passage 108 extends through the proximal collar segment 104 and the distal segment 106, and is normally biased towards a closed condition, which is shown in FIG. 2. In the closed condition, the passage 108 provides a substantially fluid-tight seal, and thus, substantially prevents the communication of fluid through the valve 102 in the absence of the surgical object "I." Additionally, the configuration of the passage 108 may assist in minimizing the escape of fluid through the valve assembly 100 when the surgical object "I" and/or surgical filaments "F" are inserted therethrough. Although depicted as including a plurality of intersecting slits 110 in the embodiment of the valve assembly 100 shown in FIG. 2, the passage 108 may be configured in any manner suitable for the intended purpose of substantially limiting the communication of fluids, e.g., insufflation gas or saline, through the valve assembly 100. As an illustrative example, the passage 108 may be configured as a single-slit valve 112 (FIG. 3).

The distal segment 106 of the valve 102 includes an outer wall 114 that inwardly tapers in a distal direction. As will be discussed in further detail below, the tapered configuration of the distal segment 106 and the fluid resistive shelf 107 cooperate to substantially minimize the egress, communication, or escape of fluid through the valve assembly 100, and thus, the establishment and maintenance of a substantially fluid tight seal. In the embodiments of the valve assembly 100 seen in FIGS. 2-6, the outer wall 114 extends in a substantially linear fashion such that the distal segment 106 defines a generally frusto-conical configuration. Alternatively, however, the outer wall 114 may be substantially arcuate.

With reference now to FIGS. 1-2 and FIGS. 4-6, the use and function of the surgical access device 1000 (FIG. 1) will be discussed during the course of a surgical procedure performed with the surgical object "I." Initially, the access member 1006 is positioned within the tissue tract 10 formed in the patient's tissue "T", and a fluid, e.g., an insufflation gas, is introduced into the surgical worksite "W" through the surgical access device 1000. As previously discussed, the passage 108 of the valve 102 is biased towards a closed position (FIG. 2), thus establishing a substantially fluid-tight seal and substantially preventing the escape of any fluid through the valve 102 prior to insertion of the surgical object "I."

As the fluid fills the surgical worksite "W", the fluid exerts pressure, represented generally by the reference character "P" (FIG. 2), on the valve assembly 100 as it tries to escape proximally through the surgical access device 1000. Specifically, the pressure "P" acts on the outer wall 114 of the distal segment 106 and the fluid resistive shelf 107 of the valve 102. The inward distal tapering of the outer wall 114 results in a component of the pressure "P" being directed inwardly in the direction of arrows 1 (FIG. 2), thus compressing the distal segment 106 of the valve 102 and assisting in the maintenance of the substantially fluid-tight seal established by the passage 108.

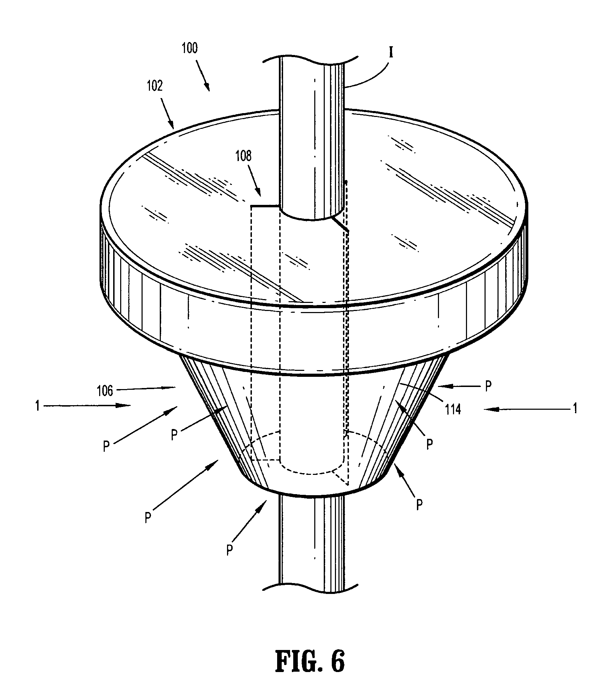

Following insufflation of the surgical worksite "W", the practitioner inserts the surgical object "I" through the surgical access device 1000, as seen in FIG. 6. As the surgical object "I" is advanced distally through the valve assembly 100, the passage 108 is forced open. However, the bias created by the resilient material comprising the valve 102 forces the passage 108 into engagement with the surgical object "I" such that communication of fluid through the valve 102 is substantially inhibited. Additionally, the pressure "P" acting on the outer wall 114 of the distal segment 106 continues to compress and force the distal segment 106 inwardly in the direction of arrows 1 and into engagement with the surgical object "I", thereby assisiting in the establishment and maintenance of a substantially fluid-tight seal between the surgical object "I" and the valve 102. The practitioner can then manipulate the surgical object "I" through the surgical access device 1000 to carry out the remainder of the procedure.

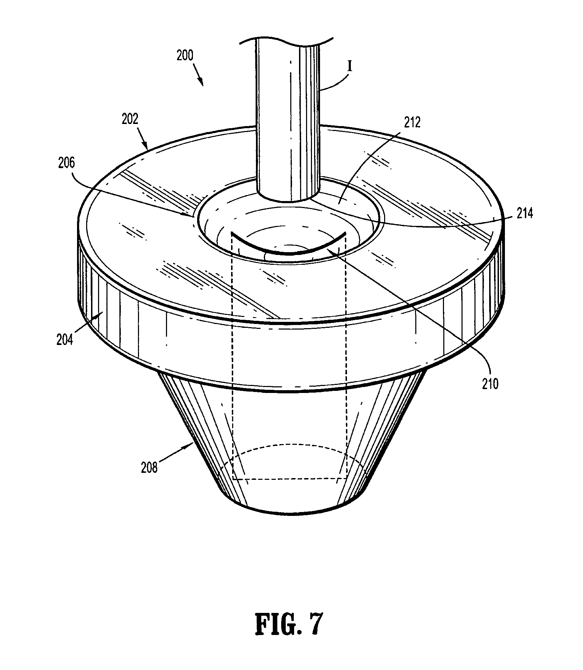

FIG. 7 illustrates an alternative embodiment of the valve assembly, referred to generally by reference character 200, including a valve 202. The valve 202 is substantially identical to the valve 102 discussed above with respect to FIGS. 1-6, and accordingly, will only be discussed with respect to its differences therefrom.

The valve 202 includes a proximal collar segment 204 with a recessed portion 206, a distal segment 208, and a passage 210. The passage 210 is formed in a concave internal wall 212 defined by the recessed portion 206, and extends through the valve 202. The concave configuration of the internal wall 212 facilitates insertion of the surgical object "I" through the valve assembly 200. More specifically, upon the introduction of the surgical object "I" to the valve assembly 200, a distal end 214 of the surgical object "I" engages the concave internal wall 212. The contour of the concave internal wall 212 guides the surgical object "I" towards the passage 210. For example, a surgical object "I" inserted into the surgical access device 1000 (FIG. 1) including valve 202 in an off-axis orientation, i.e., such that the surgical object "I" forms an angle with the longitudinal axis "A", would be urged into a substantially vertical orientation upon engagement with the concave internal wall 212 of the valve 202. As the surgical object "I" is passed through the valve assembly 200, the concave configuration of the wall 212 reduces friction between the surgical object "I" and the valve 202, thus reducing the force necessary to advance the surgical object "I" through the valve assembly 200 and further facilitating insertion of the surgical object "I." Additionally, reducing friction between the surgical object "I" and the valve 202 also reduces "spurting" of fluid through the valve 202.

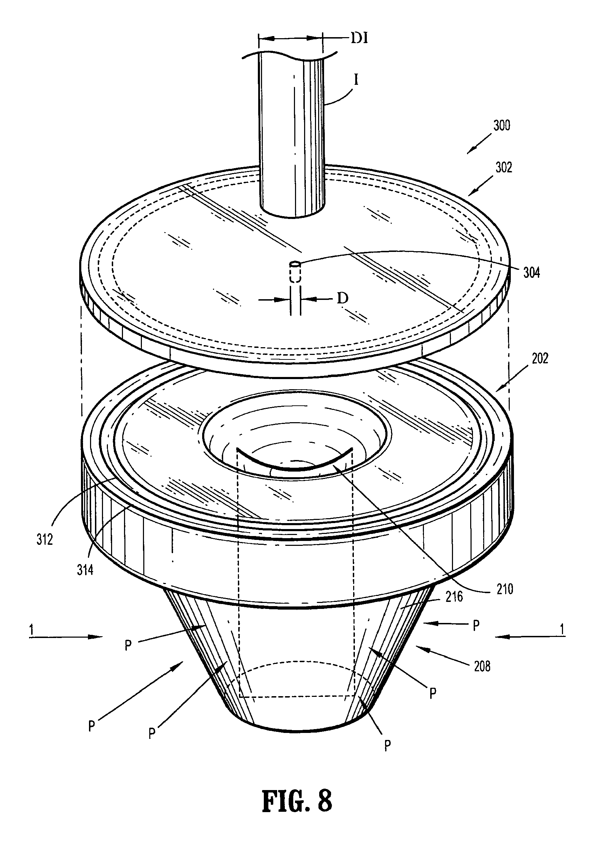

FIGS. 8-9 illustrate another embodiment of the valve assembly, referred to generally by reference character 300,including a fluid valve 202 and an instrument seal 302. Although illustrated as the valve 202 (FIG. 7), in alternative embodiments of the valve assembly 300, it is envisioned that the valve 102 (FIGS. 1-6) may be employed as the fluid valve component of the valve assembly 300.

The instrument seal 302 may be formed of any suitable material that is at least semi-resilient in nature, acceptable examples of which were discussed above with respect to the valve 102 shown in FIGS. 1-6. Forming the instrument seal 302 from such materials permits the instrument seal 302 to resiliently accommodate the insertion, manipulation, and removal of surgical instrumentation that may vary in size, e.g., outer dimensions.

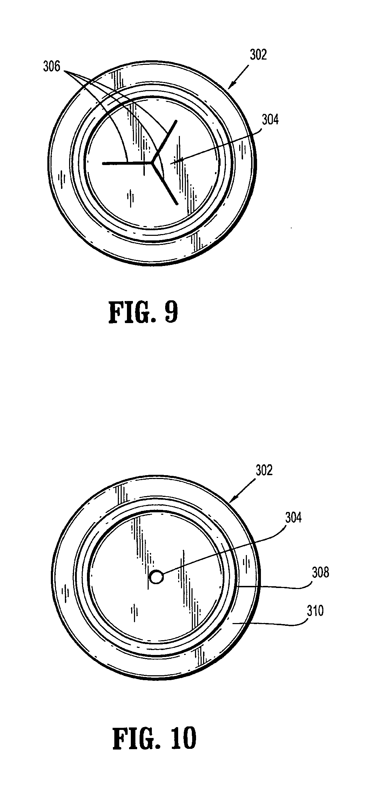

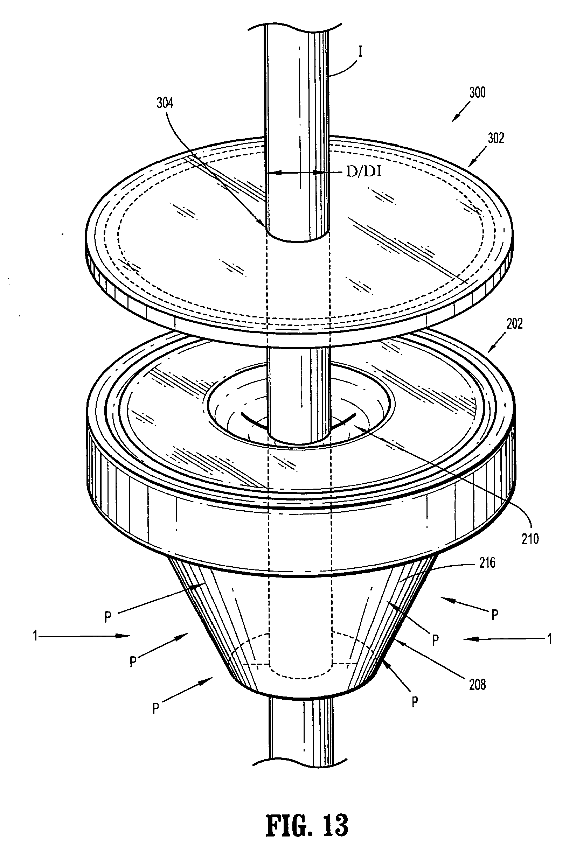

The instrument seal 302 includes a seal aperture 304 extending therethrough. The seal aperture 304 is normally biased towards a closed condition, seen in FIG. 8, in which the seal aperture 304 defines a transverse dimension "D" that is substantially smaller than an outer dimension "DI" defined by the surgical object "I." Accordingly, upon insertion of the surgical object "I" through the instrument seal 302 (FIG. 13), the seal aperture 304 is enlarged to substantially approximate the outer dimension "DI" of the surgical object "I," thereby establishing a substantially fluid-tight seal between the surgical object "I" and the instrument seal 302 and substantially preventing the communication of fluid, such as insufflation gas, through the instrument seal 302. While the seal aperture 304 is illustrated as defining a substantially annular opening, in alternate embodiments of the valve assembly 300, the instrument seal 302 may include any valve member suitable for the intended purpose of receiving the surgical object "I" such that a substantially fluid-tight seal is formed therewith. For example, the seal aperture 304 may include a plurality of intersecting slits 306, as seen in FIG. 9, a single-slit (not shown), as discussed above with respect to FIG. 3.

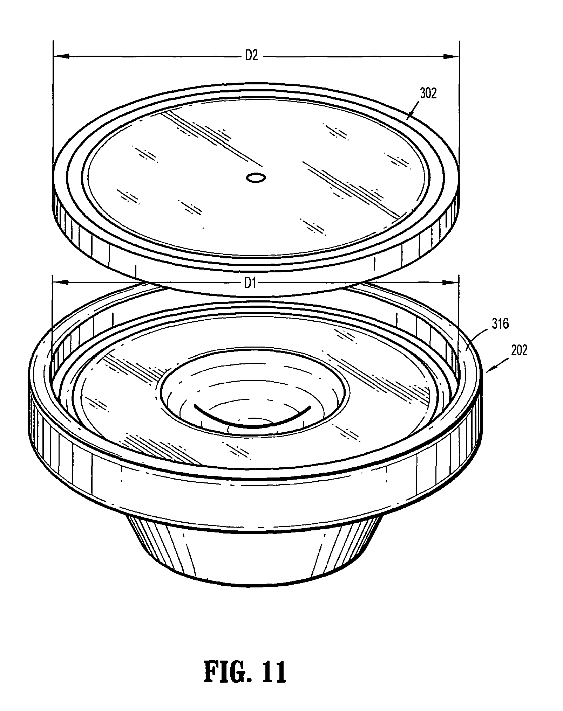

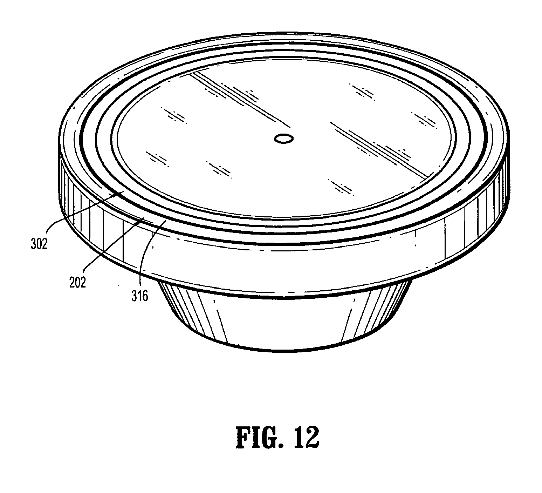

When the valve assembly 300 is disposed within the housing 1002 of the surgical access device 1000 (FIG. 1), the instrument seal 302 is positioned proximally of the valve 202. In one embodiment of the valve assembly 300, one or both of the valve 202 and the instrument seal 302 may include structure adapted to maintain the position of the instrument seal 302 relative to the valve 202. As an example, the instrument seal 302 may include a ridge 308 (FIG. 10) formed on a distal surface 310 that is configured and dimensioned to engage a corresponding recess 312 (FIG, 8) formed in a proximal surface 314 of the valve 202. Additionally, or alternatively, the valve 202 may include a raised lip 316 (FIG. 11) defining an internal dimension "D1" that substantially approximates an outer dimension "D2" of the instrument seal 302 such that the valve 202 and the instrument seal 302 may be assembled as shown in FIG. 12, i.e., with the instrument seal 302 at least partially positioned within the valve 202.

With reference now to FIGS. 1, 8-9, and 12, the use and function of the valve assembly 300 will be discussed during the course of a surgical procedure performed with the surgical object "I" in connection with the surgical access device 1000 shown in FIG. 1. Following placement of the access member 1006 within the tissue tract 10 formed in the patient's tissue "T", a fluid, such as an insufflation gas, is introduced into the surgical worksite "W" through surgical access device 1000. As previously discussed, the respective passages 210, 304 of the valve 202 and the instrument seal 302, respectively, are biased towards the closed positions seen in FIG. 8. 2), thus establishing a substantially fluid-tight seal and substantially preventing the escape of any fluid through the valve assembly 300 prior to insertion of the surgical object "I."

As the fluid fills the surgical worksite "W", the fluid exerts pressure "P" on the valve assembly 300 as the fluid tries to escape proximally through the surgical access device 1000 (FIG. 1). Specifically, the pressure "P" acts on the outer wall 216 of the distal segment 208 of the valve 202. The distal tapering of the outer wall 216 directs a component of the pressure "P" inwardly in the direction of arrows 1, thus compressing the distal segment 208 of the valve 202 and assisting in the maintenance of the substantially fluid-tight seal established by the passage 210.

Following insufflation of the surgical worksite "W", the practitioner inserts the surgical object "I" through the surgical access device 1000, as seen in FIG. 12. As the surgical object "I" is advanced distally through the valve assembly 100, the seal aperture 304 of the instrument seal 302 is enlarged, and the passage 210 of the valve 202 is forced open. However, the resilient nature of the material comprising the valve 202 and the instrument seal 302 allows the valve 202 and the instrument seal 304 to substantially approximate the outer dimension "DI" of the surgical object "I" such that the substantially fluid-tight seal established prior to the insertion of the surgical object "I" is maintained. Additionally, as previously described, the pressure "P" acting on the outer wall 216 of the distal segment 208 of the valve 202 continues to compress and force the distal segment 208 inwardly in the direction of arrows 1 and into engagement with the surgical object "I", thereby further ensuring the maintenance of the substantially fluid-tight seal formed between the surgical object "I" and the valve assembly 300. The practitioner can then manipulate the surgical object "I" through the surgical access device 1000 to carry out the remainder of the procedure.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure. For example, although the valve members discussed above have been illustrated as substantially circular in configuration, the valve members may exhibit any suitable geometrical configuration. Additionally, persons skilled in the art will appreciate that the features illustrated or described in connection with one embodiment may be combined with those of another, and that such modifications and variations are also intended to be included within the scope of the present disclosure.

## Claims

1. A surgical access device for use during a surgical procedure, comprising:
a housing;
an access member extending distally from the housing and being dimensioned for positioning within tissue, the access member defining a longitudinal axis and a longitudinal opening, and having proximal and distal ends; and
a valve at least partially disposed within the housing and having a passage for reception of a surgical object, the valve including:
a proximal collar segment;
a distal tapered segment extending contiguously from the proximal collar segment; and
a fluid resistive shelf at a juncture defined by the proximal collar segment and the distal tapered segment;
wherein at least the fluid resistive shelf and the distal tapered segment cooperate to substantially minimize egress of fluids from the operative site about the valve.

2. The surgical access device of claim 1 wherein the valve is dimensioned whereby the passage is substantially closed in the absence of the surgical object.

3. The surgical access device of claim 1 or 2 wherein the distal tapered segment of the valve defines a substantially frusto-conical shape.

4. The surgical access device of any of claims 1 to 3 wherein the valve includes at least one internal slit, the at least one internal slit defining the passage

5. The surgical access device of claim 4 wherein the valve includes a plurality of intersecting slits, the slits defining the passage

6. The surgical access device of any of claims 1 to 5 wherein the proximal collar segment of the valve includes a recessed portion defining an internal wall, the internal wall tapering radially inwardly from proximal to distal to assist in guiding the surgical object towards the passage of the valve.

7. The surgical access device of any of claims 1 to 6 wherein the valve is a solid member.

8. The surgical access device of any of claims 1 to 7 including an instrument seal mounted relative to the housing, the instrument seal defining a seal aperture for reception of the surgical object in substantial sealed relation therewith.

9. The surgical access device of claim 8 wherein at least one of the instrument seal and the valve includes structure configured and dimensioned to maintain the relative position of the instrument seal and the valve.

10. The surgical access device of claim 9 wherein the valve includes a recess configured and dimensioned to engage a corresponding ridge formed on the instrument seal.

11. The surgical access device of any of claims 1 to 10 wherein the valve includes a lip configured and dimensioned to accommodate the instrument seal such that the instrument seal is at least partially positionable within the valve.

12. A surgical access device including an access member defining a longitudinal opening and having proximal and distal ends, and a valve including a proximal collar segment, a distal tapered segment extending contiguously from the proximal collar segment, and a fluid resistive shelf at a juncture defined by the proximal collar segment and the distal tapered segment, for use in a surgical procedure wherein the procedure comprises the steps of:
accessing an operative site with the surgical access device,
introducing fluids into the operative site;
substantially minimizing egress of fluids from the operative site and about the valve through engagement of the fluids with the fluid resistive shelf and the distal tapered segment; and
introducing a surgical object through the valve and through the longitudinal opening of the access member to perform a surgical task.

13. The surgical access device of claim 12 wherein the proximal collar segment of the valve includes a recessed portion defining an internal wall tapering radially inwardly from proximal to distal and wherein the step of accessing includes guiding the surgical object towards the passage of the valve.

14. The surgical access device of claim 12 or 13 wherein the access device further includes an object seal mounted relative to the housing and wherein the step of introducing includes establishing a substantial sealed relation about the surgical object with the object seal.
